# EUROPEAN PATENT APPLICATION

(11) **EP 1 700 616 A1**
(43) Date of publication of application: **13.09.2006**
(21) Application number: 04805092.6
(22) Date of filing: 09.12.2004
(51) Int. Cl.: A61P 27/02, A61K 9/08

(54) **COMPOUNDS FOR THE TREATMENT OF OCULAR DRYNESS CAUSED BY PHOTOREFRACTIVE SURGERY**

(30) Priority: 09.12.2003 ES 200302898
(71) Applicant: Universidad Miguel Hernandez de Elche, 03202 Elche (Alicante) (ES)
(72) Inventor: BELMONTE MARTINEZ, Carlos, Elche E-Alicante (ES); GALLAR MARTINEZ, Juana, Elche E-Alicante (ES); ACOSTA BOJ, M Carmen, Elche E-Alicante (ES)
(74) Representative: Elzaburu Marquez, Alberto
(86) International application number: PCT/ES2004/000549
(87) International publication number: WO 2005/056113

(57) **Abstract**

The blocking agents of the electrical activity of the damaged nerve endings of the neuroma can be used for the treatment of dryness of the ocular surface caused by photorefractive surgery, such as excimer laser photorefractive keratectomy or laser-assisted in situ keratomileusis. The administration of said blocking agents, which include antiepileptics, anticonvulsants, anti-arrhythmic drugs, tricyclic antidepressants and local anaesthetics and, in particular, include lidocaine, tocainide, phenytoin, carbamazepine, lamotrigine, mexiletine and pregabalin, effectively reduce the sensations of ocular dryness.

## Description

This invention relates to the field of human and veterinary medicine, and specifically to compounds for the treatment of eye diseases.

### PRIOR ART

Photorefractive surgery is a surgical procedure used for correcting defects of refraction in the human eye based on altering the shape of the anterior surface of the cornea. Excimer laser photorefractive keratectomy (PRK) and laser in situ keratomileusis (LASIK) are the procedures most commonly used today for correcting myopia. Symptoms of dry eye are the most frequent among the subjective long-term problems described by patients who have undergone this type of surgery. A study of 231 PRK patients and 550 LASIK patients carried out by Hovanesian (cf. Hovanesian JA, Shah SS, Maloney RK. (2001) Symptoms of dry eye and recurrent erosion syndrome after refractive surgery. J. Cataract Refract. Surg. 27: 577-584) showed an incidence of symptoms of dry eye in 43% and 48% of these patients respectively, appearing primarily on waking in the morning.

The high incidence of subjective symptoms of dry eye in patients who have undergone LASIK surgery has been interpreted as being due to a decrease in reflex lacrimal secretion in these patients. Various authors have measured tear production and the rate of lacrimal clearance after LASIK (cf. Benitez-del-Castillo JM, del Rio T, Iradier T, Hernandez JL, Castillo A and Garcia-Sanchez J. (2001) Decrease in tear secretion and corneal sensitivity after laser in situ keratomileusis. Cornea 20:30-32. Toda I, Asano-Kato N, Komai-Hori Y, Tsubota K. (2001) Dry eye after laser in situ keratomileusis. Am J Ophthalmol. 132:1-7).

In all cases a slight decrease in tear production was observed, but the stability of the tear film remained unchanged. It is generally thought that basal lacrimal secretion is maintained in part by nerve impulses originating in the nerve endings that innervate the ocular surface (cf. Lamberts DW, Foster CS, Perry HD.(1979) Schirmer test after topical anesthesia and the tear meniscus height in normal eyes. Arch Ophthalmol. 97: 1082-1085). When these tissues are stimulated by irritating stimuli, the frequency of nerve impulses to the brain increases markedly and they produce a reflex increase in lacrimal secretion. The structures of the ocular surface and the main lacrimal gland may thus be said to form a reflex functional unit with the neural structures that interconnect them (cf. Stern ME, Gao J, Siemasko KF, Beuerman RW, Pflugfelder SC. (2004) The role of the lacrimal functional unit in the pathophysiology of dry eye. Exp Eye Res. 78:409-416).

It is generally assumed that the denervation produced by photorefractive surgery reduces corneal sensation and, in consequence, reflex tear secretion. Accordingly, the dry eye symptoms observed in patients who have undergone this surgery have been attributed to reduced tear secretion caused by a decrease in general nervous activity resulting from nerve damage in the cornea (cf. Patel S, Perez-Santonja JJ, Alio JL, Murphy PJ.(2001) Corneal sensitivity and some properties of the tear film after laser in situ keratomileusis. J Refract Surg. 17:17-24).

To alleviate the aforementioned symptoms of dryness of the ocular surface caused by photorefractive surgery, the treatments that are employed at present are artificial tears, i.e. solutions containing physiological saline and a viscoelastic additive (methylcellulose, hyaluronic acid, etc.). Their aim is to moisten the ocular surface for as long as possible.

The main limitation of these treatments is their low efficacy, as they do not act directly on the causes of the problems. Furthermore they are palliative treatments, so when the treatment is suspended the symptoms will reappear.

Accordingly, it is desirable to provide therapeutic agents for the treatment of dry eye caused by photorefractive surgery.

### EXPLANATION OF THE INVENTION

The inventors found, surprisingly, that the sensations of dry eye following photorefractive surgery originate from abnormal nervous activity developed by neuromatous nerve endings and in regeneration of the corneal nerves damaged surgically. They also found that the sensations described as "dry eye" or "eye dryness" may not reflect a real dryness, but the subjective interpretation given by the patient to the sensations produced by this abnormal nervous activity, originating in the corneal nerve fibres that were damaged and are regenerating. These sensations of dryness may be triggered by other causes, such as local presence of mediators of inflammation, stimulation by blinking or a mild ocular dryness, which in normal conditions would be insufficient to activate the intact nerve endings.

These facts make the damaged nerve endings of the neuroma therapeutic targets for the treatment of dryness of the ocular surface caused by photorefractive surgery. The inventors found that agents that block the electrical activity of the damaged nerve endings of the neuroma are therapeutic agents that can be used for said treatment.

Thus, the present invention relates to the use of an agent that blocks the electrical activity of the damaged nerve endings of the neuroma for the preparation of a medicinal product for the treatment of dryness of the surface of the human eye caused by photorefractive surgery. In a particular embodiment of the invention, the photorefractive surgery is excimer laser photorefractive keratectomy or laser-assisted in situ keratomileusis. The invention also provides a method of treatment of a mammal, including a human, suffering from dryness of the ocular surface caused by photorefractive surgery, which comprises ophthalmic administration of an agent that blocks the electrical activity of the damaged nerve endings of the neuroma, together with suitable amounts of pharmaceutically acceptable excipients to constitute a topical formulation.

In this description, "agent that blocks the electrical activity of the damaged nerve endings of the neuroma" means a compound capable of preventing or reducing the development of discharges of nerve impulses in neuromas of sensory nerve fibres of the skin or other tissues, either spontaneous or evoked by mechanical, chemical or thermal stimulation, as a consequence of the blocking action of these substances on the ion channels, producing stabilization of the membrane and controlling the excitability of the neurons.

In a particular embodiment of the invention, the blocking agent is selected from those that exert their action on the voltage-dependent sodium, calcium, chlorine and potassium channels. In particular, the blocking agent is selected from the group comprising antiepileptics, anticonvulsants, anti-arrhythmic drugs, tricyclic antidepressants and local anaesthetics, and combinations thereof.

In more particular embodiments, the blocking agent is selected from the group comprising lidocaine, tocainide, n-benzyl analogues of compounds such as tocainide, mexiletine, lamotrigine, carbamazepine, phenytoin (5,5-dephenylhydantoin), amitriptyline, N-phenylethyl amitriptyline, desipramine, gabapentin, nifekalant, venlafaxine, nefazodone, pregabalin, and the pharmaceutically acceptable salts thereof (e.g. nifekalant hydrochloride). This list includes agents blocking the electrical activity of the nerve fibres in the neuroma that are well known as such, i.e. those that are marketed or have been tested as agents that block the ion channels that are active in neuromas, but listing of the agents that are currently being marketed for this purpose is not intended to limit the range of compounds that can be used in the practical application of this invention. Any agent that blocks or attenuates the abnormal excitability of the nerve fibres that have been damaged and are regenerating can be used according to this invention.

Experimental results indicate that in the days following photorefractive surgery, increased spontaneous activity and an abnormal response to mechanical and chemical stimuli are observed in the nerve fibres innervating the damaged cornea, and that this nervous activity is similar to that evoked by dryness of the ocular surface in the normal eye. The blocking agents of the present invention attenuate the abnormal activity in the nerves of the cornea after surgery and reduce the symptoms of ocular dryness in patients undergoing photorefractive surgery.

The invention also provides a pharmaceutical composition for ophthalmic application that comprises a therapeutically effective amount of a blocking agent such as those described above, together with suitable amounts of pharmaceutically acceptable excipients for constituting an ophthalmic formulation.

Depending on circumstances, a person skilled in the art will select a release system that is suitable for ophthalmic administration of these compositions. The blocking activity of the agents stated will vary considerably from one compound to another. The effective dose, when we are considering topical administration for treating dry eye, is also subject to a wide range of variation with respect to its therapeutic efficacy. This value will be determined by a number of factors, including the inherent activity of the drug in question, the vehicle in which it is administered, the size of the area to be treated, its penetration and bioavailability and the intensity of the sensation. The ability to determine the effective dose for any of the compounds selected is within the capabilities of a person skilled in the art. For ophthalmic application, the preferred solutions are prepared in such a way that the blocking agent is in an amount between 0.0005 and 1% (w/v), and more particularly between 0.0005 and 0.1%, in a saline solution as principal vehicle.

In the practical application of this invention, the blocking agents are administered in such a way that the drug is applied directly to the place where the abnormal electrical activity is generated. It is expected that this would be the immediate area of the lesion. For example, the drug could be applied topically, or by some similar means that applies the drug directly on the affected area of the ocular surface. It is not intended that this invention should be carried out by administering the drug in such a way as to ensure that it reaches the central nervous system. In fact, that would defeat the purpose of this invention, which focuses on treating the problem at its place of origin.

The pH of these ophthalmic solutions should preferably be maintained between 6.5 and 7.2 with a suitable buffer. The formulations can also contain conventional, pharmaceutically acceptable preservatives, stabilizers and/or penetration enhancers as well as viscoelastic substances included in artificial tear preparations.

The preferred vehicle for use in the ophthalmic solutions of the present invention is purified water and preferably a physiological saline solution. Other additional vehicles include, but are not restricted to, viscous agents such as polyvinyl alcohol, povidone; hydroxypropylmethylcellulose, poloxamers, carboxymethylcellulose, carbomers, hydroxyethylcellulose, hyaluronic acid and their derivatives. The preferred preservatives for use in the ophthalmic formulations of the present invention include, but are not restricted to, benzalkonium chloride, chlorobutanol, thimerosal, phenylmercury acetate and phenylmercury nitrate.

Penetration enhancers can be, for example, surfactants, certain organic solvents such as dimethylsulphoxide and other sulphoxides, dimethylacetamide and pyrrolidone; certain amides of heterocyclic amines, glycols (e.g. propyleneglycol); propylene carbonate; oleic acid; alkylamines and derivatives; various cationic, anionic and nonionic surfactants, amphoteric surfactants and the like.

Tonicity regulators will be added if necessary or appropriate. These include, but are not limited to, salts, especially sodium chloride, potassium chloride, mannitol and glycerol and any other ophthalmically acceptable tonicity regulator. It will be possible to use various buffers and media for adjusting the pH, provided the resulting preparation is ophthalmically acceptable. On that basis, the buffers will include acetate, citrate, phosphate and borate buffers for ophthalmic use. Similarly, the ophthalmically acceptable antioxidants for use in the present invention include, but are not limited to, sodium metabisulphite, sodium thiosulphate, acetylcysteine, butylated hydroxyanisole and butylated hydroxytoluene.

Other components that can be included in the ophthalmic preparations are chelating agents. The preferred chelating agent is disodium edelate but others can be used instead of or in combination with this.

Throughout the description and the claims the word "comprises" and its variants are not intended to exclude other technical characteristics, additives, components or steps. For a person skilled in the art, other aims, advantages and characteristics of the invention will emerge partly from the description and partly from practical application of the invention. With the details provided of the compositions for the topical formulations of the present invention and the specific instructions for their use in the treatment of dry eye, a person skilled in the art will have sufficient knowledge for developing other formulations and adapting the treatment (formulations, dose etc.) to a particular situation. The following particular embodiments are provided by way of illustration, and it is not intended that they should limit the present invention.

### DETAILED DESCRIPTION OF EMBODIMENTS OF THE INVENTION

### Study No. 1

A clinical study was carried out for comparing the attenuating effect produced by the topical administration of carbamazepine and of a placebo on the sensations of ocular dryness that appear after photorefractive surgery of the LASIK type. The study included 124 subjects, men and women in the age range 21-45 years, who underwent routine, elective, unilateral LASIK surgery for correction of myopia. The carbamazepine was administered in ophthalmic solution at 0.01%. After surgery, the subjects received one drop of the study medication in the operated eye, twice a day for 14 consecutive days. Postoperative examinations were carried out on days 1, 3, 7 and 14. The efficacy of the treatment was assessed by measuring the sensation of ocular dryness, pain intensity, and the overall analgesic efficacy, employing independent visual-analogy scales. The symptoms of eye inflammation, burning/stinging sensations, lacrimation etc. were also recorded.

The results of this study showed a more reduced incidence of the symptoms of ocular dryness in the patients treated with carbamazepine than in those treated with the placebo, the difference being statistically significant. The incidence of symptoms of dryness throughout the day was also significantly lower.

### Study No. 2

Photorefractive surgery of the PRK type was carried out on three adult cats anaesthetized with pentobarbital sodium (Nembutal, 40 mg/kg intraperitoneal (i.p.)). The cornea of both eyes was also anaesthetized topically by instillation of tetracaine 0.1% and oxybuprocaine hydrochloride 0.4%. The corneal epithelium was removed manually and an ablation was performed in the centre of the cornea 6 mm in diameter and 70 µm deep using a single-beam excimer laser with emission wavelength of 193 nm, pulse repetition rate 10 Hz, and radiation exposure of 180 mJ/cm².

Nerve recording experiments were carried out 12-48 hours after surgery. The animals were anaesthetized with Nembutal (40 mg/kg, i.p.) and were maintained in a state of areflexia during the experiment by intravenous infusion of dilute Nembutal (5 mg/kg) in the saphenous vein. The animals breathed spontaneously through a tracheal cannula. Respiratory CO₂, rectal temperature and arterial pressure were monitored continuously and were kept stable. The animals were sacrificed at the end of the experiment with an overdose of Nembutal.

An extracellular recording was made of isolated nerve fibres that innervate the cornea, obtained from the ciliary nerves of the eye, using silver chloride electrodes and conventional electrophysiological equipment, as described previously (cf. Belmonte, C., Gallar, J., Pozo, M.A., Rebollo, I. (1991) Excitation by irritant chemical substances of sensory afferent units in the cat's cornea. J. Physiol. 437: 709-725).

The corneal sensory fibres were identified by their response to slight mechanical stimulation with a wet brush. The mechanical threshold was measured with a Cochet-Bonnet aesthesiometer with a No. 12 filament (0.1-1.9 mN) and calibrated von Frey hairs (0.002-2.0 N). The receptor fields were mapped with a suprathreshold force hair. Sensitivity to chemical stimuli was investigated by applying a pulse of gas containing 98% CO₂ at a flow rate of 80 ml/min on the receptor field for 30 s.

The conduction velocity of the fibres recorded was measured by observing the latency to electric shocks (0.1-0.5 ms, 0.5-3.0 mA) applied on the receptor field with a pair of silver electrodes 3-5 mm apart. The conduction distance was estimated with a silk thread of 0.8 G positioned on the path of the nerve.

The nerve discharges and the stimulation pulses were recorded on an FM magnetic tape recorder for off-line computer analysis with suitable software (CED 1401plus and Spike2 for Windows). The spontaneous activity (average discharge frequency in pulses per second) and the changes in discharge caused by the various stimuli were measured. The fibres were classified as peripheral and central according to the localization of their receptor field (see Table 1). Most of the fibres recorded in the operated corneas had receptor fields inside and outside of the treated zone. Only one fibre responded to the stimulus exactly inside the lesion. Depending on the localization of the receptor field, the fibres were classified into fibres with field remote from the ablation, close to the ablation, inside and outside of the ablation and exactly inside the ablation.

The average mechanical threshold of the corneal nerve fibres was higher in the operated corneas (see Table 2). Three of the seven fibres with peripheral receptor field and thirteen of the 26 that had a central field displayed a mechanical stimulus threshold out of range in the operated zone. In percentage terms, the number of fibres with spontaneous activity was significantly higher in the operated corneas. So too was the average frequency of this spontaneous activity both in the fibres with receptor field in the central operated zone and at the periphery (see Table 1).

The response of the polymodal nociceptors to stimulation with CO₂ was investigated in fibres with the receptor field inside, on the edge and remote from the lesion. The response of all of them to stimulation with CO₂ was of lower amplitude and greater latency than in the fibres of the unoperated corneas, especially when the zone of the receptor field localized in the operated zone was stimulated. Moreover, the postdischarge evoked by stimulation with CO₂ was significantly greater in the fibres that innervate the operated corneas (see Table 3).

The results show that 24-48 hours after PRK the nerve fibres that innervate the damaged zone exhibit marked spontaneous activity and an abnormal response to mechanical and chemical stimuli, particularly with respect to their sustained postdischarge. Fibres with a receptor field remote from the wound react similarly to those of the control corneas (cf. Chen, X., Gallar, J. , Pozo, M.A., Baeza, M. and Belmonte, C. (1995) CO2 stimulation of the cornea: A comparison between human sensation and nerve activity in polymodal nociceptive afferents of the cat. Eur. J. Neurosci. 7: 1154-1163) except that they have a higher postdischarge.

Taken together, these results show that the functional properties of all of the fibres in the operated corneas, with or without direct damage in their receptor field, were affected by the operation, when compared with the controls. The mechanical threshold was increased, displaying high spontaneous activity and prolonged postdischarges after stimulation.

**Table 1. Receptor fields of the corneal nociceptive fibres, recorded after PRK**

| | | nociceptive fibres |
|---|---|---|
| central cornea | inside the ablation | 1 |
| | inside and outside | 11 |
| | outside | 28 |
| peripheral cornea | | 10 |

**Table 2. Functional properties of the corneal nociceptive fibres**

| | | | |
|---|---|---|---|
| Two asterisks indicate control data of Chen, X., Gallar, J., Pozo, M.A., Baeza, M. and Belmonte, C. (1995) CO2 stimulation of the cornea: A comparison between human sensation and nerve activity in polymodal nociceptive afferents of the cat. Eur. J. Neurosci. 7: 1154-1163, which are the average of myelinic and amyelinic fibres. The symbol † indicates p<0.001, and a single asterisk indicates p<0.05 t-test. | | | |

| corneal nociceptors | central cornea | peripheral cornea | control corneas** |
|---|---|---|---|
| conduction velocity (m/s) | 3.5 ± 0.5 [0.6-14] (n=36) | 3.7 ± 1.0 [0.4-8] (n=8) | 3.6 ± 2.5 (n=69) |
| receptor field (mm) | 7.4 ± 0.6 [3-12] (n=23) | 6.5 ± 0.5 [6-7] (n=2) | 5.2 ± 0.8 (n=81) |
| spontaneous activity (imp/s) | 0.58 ± 0.18 † [0.02-1.77] (15/36) | 0.76 (1/10) | 0.14 ± 0.07 (n=17) |
| mechanical threshold (mN) | 2.17 ± 0.84 (n=13) | 2.33 ± 1.25 (n=4) | 0.78 ± 0.01 (n=65) |

**Table 3. Response of the polymodal nociceptive fibres to CO₂ applied in different areas of their receptor field. The symbol † indicates p<0.001, and an asterisk indicates p<0.05 t-test, differences from the control.**

| chemical stimulation | inside the wound | external edge of the wound | far from the wound | control corneas ** |
|---|---|---|---|---|
| latency | 11.2±2.6* n=13 | 4.6±1.8 n=20 | 6.1±2.1 n=20 | 3.9±1.6 n=17 |
| CO₂ (30s) | 0.57±0.17 † n=21 | 2.07±0.56 n=21 | 1.64±0.45 n=21 | 2.4±0.4 n=17 |
| peak frequency | 5.0±1.5* n=17 | 8.9±1.8 n=19 | 8.4±2.5 n=19 | 10.1±1.8 n=17 |
| time at peak | 9.2±2.5* n=17 | 7.2±1.9* n=20 | 6.5±2.0 n=20 | 2.7±0.4 n=17 |
| postdischarge | 0.15±0.05 n=21 | 0.47±0.25 n=21 | 0.49±0.14* n=21 | 0.08±0.03 n=17 |

### Study No. 3

Tests were carried out on fifteen adult cats of both sexes, anaesthetized with pentobarbital sodium (Nembutal, 40 mg/kg, i.p.). The cornea of both eyes was anaesthetized topically by instillation of tetracaine 0.1% and oxybuprocaine hydrochloride 0.4%.

A circular wound was made in the centre of the cornea with a 5 mm dia. trepan which penetrated 40-50 µm below the Bowman membrane. The animals were treated with topical and systemic antibiotics, leaving them to recover for 1-2 weeks.

The recording tests were carried out 7 and 15 days after surgery. The animals were anaesthetized with Nembutal (40 mg/kg, i.p.) and were maintained in a state of areflexia during the experiment by intravenous infusion of dilute Nembutal (5 mg/kg) in the saphenous vein. The animals breathed spontaneously through a tracheal cannula. Respiratory CO₂, rectal temperature and arterial pressure were monitored continuously and were kept stable. The animals were sacrificed with an overdose of Nembutal at the end of the experiment.

Extracellular recording was made of nerve fibres isolated from the cornea, obtained from the ciliary nerves of the eye, using silver/silver chloride electrodes and conventional electrophysiological equipment, as described previously (cf. Belmonte, C., Gallar, J., Pozo, M.A., Rebollo, I. (1991) Excitation by irritant chemical substances of sensory afferent units in the cat's cornea. J. Physiol. 437: 709-725).

The corneal sensory fibres were identified by their response to mild stimulation with a wet brush. The mechanical threshold was measured with a Cochet-Bonnet aesthesiometer with a No. 12 filament (0.1-1.9 mN) and with calibrated von Frey hairs (0.002-2.0 N). The receptor fields were mapped with a suprathreshold force hair. Sensitivity to chemical stimuli was investigated by applying a pulse of gas containing 98% CO₂ at a flow rate of 80 ml/min on the receptor field for 30 s.

The conduction velocity was measured by observing the latency to electric shocks (0.1-0.5 ms, 0.5-3.0 mA) applied on the receptor field with a pair of silver electrodes 3-5 mm apart. The conduction distance was estimated with a silk thread of 0.8 G positioned on the path of the nerve.

The nerve discharges and the stimulation pulses were recorded on an FM magnetic tape recorder for off-line computer analysis with suitable software (CED 1401plus and Spike2 for Windows). The spontaneous activity (average discharge frequency in pulses per second) and the changes in discharge caused by the various stimuli were measured.

The fibres were classified as peripheral and central according to the localization of their receptor field. Most of the nociceptive units recorded in the damaged corneas had receptor fields both outside of the wound and on its edge. Depending on the localization of the receptor field the fibres were classified as far from the wound or close to or bordering on the wound. The control data were obtained from intact corneas.

The frequency of spontaneous discharge of the fibres innervating the cornea was greater in the damaged corneas than in the intact corneas. The response of the polymodal nociceptive fibres to chemical stimulation was investigated in the fibres whose receptor field touched the damaged area and in those whose receptor field was outside of and far from the lesion. The spontaneous activity and the discharge of the fibres in response to stimulation with CO₂ were measured before and after topical application of carbamazepine (0.001-0.01%), phenytoin (5,5-diphenylhydantoin) (0.001-0.1%), mexiletine (0.001-0.1%), lidocaine (0.0005-0.01%) and tocaidine (0.001-0.01%). All these substances produced a dose-dependent decrease in spontaneous activity of the fibres innervating the zones around the lesion and the responses evoked by pulses of CO₂ of 30 s duration to these areas.

## Claims

1. Use of a blocking agent of the electrical activity of the damaged nerve endings of the neuroma for the preparation of a medicinal product for the treatment of dryness of the surface of the human eye caused by photorefractive surgery.

2. Use according to claim 1, in which the photorefractive surgery is an excimer laser photorefractive keratectomy or a laser-assisted in situ keratomileusis.

3. Use according to any one of the preceding claims, **characterized in that** the blocking agent is selected from those that exert their action on the voltage-dependent sodium, calcium, chlorine and potassium channels.

4. Use according to any one of the preceding claims, **characterized in that** the blocking agent is selected from the group comprising antiepileptics, anticonvulsants, anti-arrhythmic drugs, tricyclic antidepressants and local anaesthetics, and combinations thereof.

5. Use according to claim 4, **characterized in that** the blocking agent is selected from the group comprising lidocaine, tocainide, n-benzyl analogues of compounds such as tocainide, mexiletine, lamotrigine, carbamazepine, phenytoin, amitriptyline, N-phenylethyl amitriptyline, desipramine, gabapentin, nifekalant, venlafaxine, nefazodone, pregabalin, and the pharmaceutically acceptable salts thereof.

6. Use according to claim 5, **characterized in that** the blocking agent is carbamazepine.

7. Use according to claim 5, **characterized in that** the blocking agent is phenytoin.

8. Use according to claim 5, **characterized in that** the blocking agent is mexiletine.

9. Use according to claim 5, **characterized in that** the blocking agent is lidocaine.

10. Use according to claim 5, **characterized in that** the blocking agent is tocaidine.

11. Use according to claim 5, **characterized in that** the blocking agent is pregabalin.

12. Pharmaceutical composition for ophthalmic application that comprises a therapeutically effective amount of a blocking agent as described in any one of the preceding claims, together with suitable amounts of pharmaceutically acceptable excipients for constituting an ophthalmic formulation.

13. Composition according to claim 12, **characterized in that** the blocking agent is in an amount between 0.0005 and 1% (w/v).

14. Composition according to claim 13, **characterized in that** the blocking agent is in an amount between 0.0005 and 0.1% (w/v).

15. Method of treatment of a mammal, including a human, suffering from dryness of the ocular surface caused by photorefractive surgery, which comprises the ophthalmic administration of an agent for blocking the electrical activity of the damaged nerve endings of the neuroma, together with suitable amounts of pharmaceutically acceptable excipients for constituting a topical formulation.

16. Method according to claim 15, **characterized in that** the photorefractive surgery is an excimer laser photorefractive keratectomy or a laser-assisted in situ keratomileusis.

17. Method according to any one of the claims 15-16, **characterized in that** the blocking agent is selected from those that exert their action on the voltage-dependent sodium, calcium, chlorine and potassium channels.

18. Method according to any one of the claims 15-17, **characterized in that** the blocking agent is selected from the group comprising antiepileptics, anticonvulsants, anti-arrhythmic drugs, tricyclic antidepressants and local anaesthetics, and combinations thereof.

19. Method according to claim 18, **characterized in that** the blocking agent is selected from the group comprising lidocaine, tocainide, n-benzyl analogues of compounds such as tocainide, mexiletine, lamotrigine, carbamazepine, phenytoin, amitriptyline, N-phenylethyl amitriptyline, desipramine, gabapentin, nifekalant, venlafaxine, nefazodone, pregabalin, and the pharmaceutically acceptable salts thereof.

20. Method according to claim 19, **characterized in that** the blocking agent is carbamazepine.

21. Method according to claim 19, **characterized in that** the blocking agent is phenytoin.

22. Method according to claim 19, **characterized in that** the blocking agent is mexiletine.

23. Method according to claim 19, **characterized in that** the blocking agent is lidocaine.

24. Method according to claim 19, **characterized in that** the blocking agent is tocaidine.

25. Method according to claim 19, **characterized in that** the blocking agent is pregabalin.
